# EUROPEAN PATENT SPECIFICATION

(11) **EP 1 066 015 B1**
(45) Date of publication and mention of the grant of the patent: **11.02.2004**
(21) Application number: 98938990.3
(22) Date of filing: 31.07.1998
(51) Int. Cl.: A61K 7/06

(54) **TOPICAL LIQUID COMPOSITION FOR PROMOTING HAIR GROWTH AND PROCESS OF MANUFACTURING THEREOF**
TOPISCHE FLÜSSIGE ZUBEREITUNG ZUR FÖRDERUNG DES HAARWUCHSES UND VERFAHREN ZU IHRER HERSTELLUNG
COMPOSITION TOPIQUE LIQUIDE FAVORISANT LA POUSSE DES CHEVEUX ET SON PROCEDE DE FABRICATION

(30) Priority: 01.04.1998 KR 9811521
(43) Date of publication of application: 10.01.2001
(73) Proprietor: Spela Co., Ltd., Seoul (KR)
(72) Inventor: Spela Co., Ltd., Seoul (KR)
(74) Representative: Weber, Dieter, Dr.
(86) International application number: PCT/KR1998/000234
(87) International publication number: WO 1999/049839

(56) References cited:
- WO-A1-97/16158
- FR-A1- 2 558 727
- PATENT ABSTRACTS OF JAPAN, Vol. 14, No. 211, (C-715), 1990; & JP 2048517 A (SHISEIDO CO.).
- PATENT ABSTRACTS OF JAPAN, Vol. 14, No. 211, (C-715), 1990; & JP 2048514 A (SHISEIDO CO.).
- PATENT ABSTRACTS OF JAPAN, Vol. 17, No. 211, (C-1052), 1993; & JP 4352710 A (FUJII K.K.).
- DATABASE WPI ON EPOQUE, week 8943, LONDON: DERWENT PUBLICATIONS LTD., AN 89-314088, Class B04; & JP 1233207 A (CHUWA INT.).
- DATABASE WPI ON EPOQUE, week 8537, LONDON: DERWENT PUBLICATIONS LTD., AN 85-226622, Class B04; & JP 60146829 A (ROHTO).

## Description

### BACKGROUND OF THE INVENTION

### Field of the Invention

The invention herein relates to a topical liquid composition for promoting hair growth, comprising natural ingredients with no side-effects, which is effective in promoting hair growth and preventing hair loss. More particularly, the invention relates to the topical liquid composition for promoting hair growth, comprising the extracts which are formed by immersing the mixture of powders of Pinelliae Rhizoma, Caryophylli Flos, Rubi Fructus, Zanthoxyli Fructus, Viticis Fructus, Salviae Radix, and Thujae Semen in oil and ethanol, respectively, followed by aging for a fixed period of time.

### Description of the Prior Art

Hair has a life span of a sort as hair continuously falls out, and new hairs begin to grow. In general, alopecia denotes a loss or absence of hair and typically includes alopecia areata. It is physiological in a normal sense that a person should shed 70 to 80 hairs a day. Alopecia also includes the balding from excessive hair loss and thinning, in whole or in part. In the case of severe alopecia, a patient should consult a dermatologist since it may be a systemic disease.

As for the forms of alopecia, they could be congenital due to hereditary cause or acquired symptoms from various environmental factors. As for the direct causes, they include excessive stress, tension on the scalp muscle, excessive secretion of male hormone, androgen, or relative reduction in secretion of female hormone, seborrheic dermatitis and atopic constitution, and physical stimulation. In particular, excessive intake of fat and exposure to pollution have been recognized as major causes of alopecia.

Presently, several methods have been disclosed for the prevention and treatment of aforementioned alopecia. For example, Korean Unexamined Patent No. 91-106 discloses a method of using a rhododendron extract as active ingredient. Korean Unexamined Patent No. 96-40346 discloses a hair-growth promoter using a pine biloba extract: the Korean Unexamined Patent No. 90-64 relates to a hair-growth promoter using a quartz porphyry. Korean Unexamined Patent No. 90-2757 discloses a hair-growth promoter using a silkworm.

Nevertheless, there is a great need for further improving the shortcomings of the conventional methods since they do not provide expected therapeutic effectiveness in promoting hair growth and preventing hair loss.

### SUMMAY OF THE INVENTION

The objective of the invention herein is to provide a topical liquid composition for promoting hair growth, comprising of natural ingredients with no side-effects, which is effective in promoting hair growth and preventing hair loss, by means of immersing various natural ingredients in oil and ethanol, respectively, followed by aging for a fixed period of time.

### Detailed Description of the Invention

This invention relates to a topical liquid composition for promoting hair growth comprising extracts of Pinelliae Rhizoma, Caryophylli Flos, Rubi Fructus, Zanthoxyli Fructus, Viticis Fructus, Salviae Radix, and Thujae Semen.

In addition, the invention relates to a process of preparing the topical liquid composition for promoting hair growth, wherein 20 - 40 weigh parts of a mixture of respective powders of Pinelliae Rhizoma, Caryophylli Flos, Rubi Fructus, Zanthoxyli Fructus, Viticis Fructus, Salviae Radix, and Thujae Semen is immersed in 100 weight parts of oil, and 100 weight parts of ethanol. Then, the oil and ethanol extracts, so obtained via aging for 5 - 10 days, are blended in the respective weight ratio of 1 : 10 - 15.

The invention is explained in more detail as set forth hereunder. The invention relates to a topical liquid composition for promoting hair growth, comprising a mixture of extracts of Pinelliae Rhizoma, Caryophylli Flos, Rubi Fructus, Zanthoxyli Fructus, Viticis Fructus, Salviae Radix, and Thujae Semen, obtained by means of immersing said ingredients in oil and ethanol, respectively.

The characteristics of each active ingredient according to the manufacturing process are provided in more detail as set forth hereunder. According to this invention, Pinelliae Rhizoma, Caryophylli Flos, Rubi Fructus, Zanthoxyli Fructus, Viticis Fructus, Salviae Radix, and Thujae Semen is prepared in the form of powder.

The detailed description of each active ingredient are as follows:
Pinelliae Rhizoma is a tuber after removing the cork layer of Pinellia ternata Breitenbach (Araceae), which is distributed throughout Korea. There are various preparations of Pinelliae Rhizoma. For example, the fresh Pinelliae Rhizoma is gathered in the summer and dried in the sun. The blue Pinelliae Rhizoma is prepared with boiled rice, and the Kang-Pinelliae Rhizoma is prepared with ginger and boiled rice. The Pup-Pinelliae Rhizoma is packaged by adding boiled rice, lime, licorice, and ginger. The Pinelliae Rhizoma pottage is a malt prepared by adding wheat flour. red bean, and apricot stone to the Kang-Pinelliae Rhizoma in a powder. As for the indications, Pinelliae Rhizoma has the effect of drying dampness, resolving phlegm and preventing vomiting. An appropriate amount of 3 - 10g/day of softy milled crude drug has been clinically used by topical means.
Caryophylli Flos is the bud of the Caryophylli Flos plant (Eugenia caryophyllata Thumb, Myrtaceae), which is customarily called Kong Ding Xiang in China. Caryophylli Flos is mainly cultivated in Tanzania, Malaysia, Indonesia, and Guandong in China. When the green bud of Caryophylli Flos changes into red during the period from September to next March, it is carefully plucked. With the removal of a flower stalk, it is dried in the sun prior to use. Caryophylli Flos is effective for clearing away heat and toxic material, and warming the kidney. It is administered in a dose of 1 - 3g.
Rubi Fructus is an unripe fruit of Rubus Coreanus Miq. (Rosaceae). The unripe fruits are picked during the summer, placed in boiling water and dried in the sun prior to use. Rubi Fructus is known to be effective for tonifying the kidney and decreasing urination. It is administered by decoction in a dose of 3 - 10g.
Zanthoxyli Fructus is the skin of the ripe fruit of Zanthoxylum Piperitum D.C. or other members of the Rubaceae family, with the removal of the seeds. The ripe fruits are picked in autumn, dried in the sun or extracted prior to use. Zanthoxyli Fructus is effective for relieving pain, killing parasites, inhibiting cough, and soothing asthma. It is topically administered in an appropriate amount of 3 - 6g.
Viticis Fructus is a fruit of Vitex Rotundifolia L. Fil. (Verbenaceae). In general, it is picked during the summer, dried in shade and parched until turning yellow. Viticis Fructus is effective for clearing and dissipating heat, clearing the head, and brightening the eyes. It is preferable that this crude medicine be administered in a dose of 5 - 10g per day.
Salviae Radix is mainly produced in several regions of China, such as Anghui, Jiangsu, and Sichuan. Its therapeutic effects include invigorating blood, removing stagnation, cooling blood, reducing carbuncles, clearing heat in the heart, and soothing irritability. Salvia Radix usually is administered in a dose of 5 - 15g by decocting.
Lastly, Thujae Semen is the seed of Thuja Orientalis Linne (Cupressaceae). Its ripe seed is collected in the early winter. The testae of the seed is removed, and the core is dried in the sun. Thujae Semen is distributed throughout Korea and China. In particular, the main areas of production include Chechon, Choongbuk of Korea, and Shandong, Heinam, Heibei of China. Thujae Semen is good for nourishment and pacifying the liver. It is generally administered in a dose of 10 - 20g.

The invention herein is characterized in that seven different herbal components are prepared in a powder form by a bead mill in the range of 80 - 100 mesh. Then, 20 - 40 weight parts of the mixed powder is immersed in 100 weight parts of oil and 100 weight parts of ethanol for 5 - 10 days. The reason for limiting the total contents of the herbal components to 20 - 40 weight part to 100 weight parts of oil and 100 weight parts of ethanol lies in the fact that if such contents are less than 20 weight parts, the herbal extraction capacity is reduced. If it exceeds 40 weight parts for immersion, the improvement of therapeutic effect cannot be expected, and the manufacturing process is not deemed to be economical. Further, it is preferable that the blending ratios of each herbal component be in the range of 1:10 to the total extract. It is also preferable that the blending ratio be equal in weight ratio per each herbal component. It is preferable that one or more vegetable oils be blended, which are selected from the group consisting of sesame oil, perilla oil and Thujae Semen oil, and prepared by the commonly used method. In particular, it is most preferable that the blending ratio be 50% of sesame oil, 20% of perilla oil, and 30% of Thujae Semen oil by weight.

The topical solution according to this invention is prepared with the blending ratio of 1:10 - 15 between the oil extract and ethanol extract having the active ingredients. In the case of the oil extract, Caryophylli Flos and Zanthoxyli Fructus having volatile characteristics are extracted. In the case of the ethanol extract, Pinellia Rhizoma, Rubi Fructus, Viticis Fructus, Salviae Radix and Thujae Semen are extracted. The topical solution, derived from the mixture of these herbal extracts, is effective in promoting hair growth and preventing hair loss without any side-effects.

The above preparation is topically used in a liquid form, and its use is very convenient. It is preferable that the composition be applied in a dose of 1,000 - 3,000 mg per day. It is most preferable that the topical preparation be applied to the affected epidermal site for 1 - 2 times daily in a dose of 1 - 3cc.

The following examples illustrates various aspects of this invention.

### Example

First, Pinelliae Rhizoma, Caryophylli Flos, Rubi Fructus, Zanthoxyli Fructus, Viticis Fructus, Salviae Radix and Thujae Semen, respectively, were made into powder by means of meshing machine. The powders were evenly mixed in equal proportions by weight in a stirring machine.

30g of a herbal powder mixture was immersed into 100cc of vegetable oil consisting of 50% of sesame oil, 20% of perilla oil, and 30% of Thujae Semen oil by weight for 7 days. Separately, in the same procedure as described above, 30g of the herbal powder mixture was immersed into 100cc of ethanol for 7 days. Then, a mixture consisting of 5% of oil extract, 55% of ethanol extract, and 40% of purified water by weight was well blended in a stirring machine to yield a topical liquid preparation.

### Experimental example 1: Application of a topical liquid preparation to the areas of hair loss induced by drugs and/or radiological treatment

With the sample of 10 patients, the topical liquid preparations, so prepared from the example, were applied to the areas of hair loss induced by drug administration and/or radiological exposure in dose of 3cc for two times per day for the duration of 6 months. As a result, the topical liquid preparation was shown to have remarkable hair-growth effect including generation of new hair roots in 8 patients.

### Comparative experimental example 1: Application of a topical liquid preparation to the areas of hair loss induced by drugs and/or radiological treatment

The currently available drugs for hair growth are directed to the improvement of blood circulation in the surrounding area of scalp in the hair root. These include the Minoxidil agent which was approved in 1979 by the Food and Drug Administration of U.S.A. as a hair-growth agent, i.e., Baldmin® (Choong-wae Pharmaceutical Co., Ltd.), Moxidil® (Hanmi Pharmaceutical Co., Ltd.), Minoxidil® (Hyundai Pharmaceutical Co., Ltd.), Doctor Mo® (Pacific Pharmaceutical Co., Ltd.), and Grobix® (Kyungin Pharmaceutical Co. Ltd.). With the same testing condition as above, however, these preparations failed to exhibit the therapeutic effects in 10 patients with hair loss induced by the drug administration and/or radiological exposure.

### Experiment example 2: Application of a topical liquid preparation to the areas of hair loss induced by atopic dermatitis

In general, an atopic patient is vulnerable to atopic alopecia as well as atopic dermatitis. Under the same conditions as described in the example 1, the topical liquid preparation, so prepared from the example, was applied to the areas of hair loss induced by atopic dermatitis. As a result, the topical liquid preparation was effective in the treatment of atopic dermatitis and implanting new hair roots.

### Comparative experimental example 2 : Application of a topical liquid preparation to the areas of hair loss induced by atopic dermatitis

Under the same conditions as the experimental example 1, the hair-growth agents of the comparative experimental example 1 were applied to the areas of hair loss induced by atopic dermatitis. However, there were no therapeutic effects with respect to the treatment of atopic dermatitis.

### Experimental example 3: Application of a topical liquid preparation to the areas of hair loss induced by alopecia areata

Under the same conditions as described in the experimental example 1, the topical liquid preparation, so prepared from the example was applied to the areas of hair loss induced by alopecia areata. As a result, the preparation exhibited a remarkable therapeutic effect observable even with the naked eyes.

### Comparative experimental example 3 : Application of a topical liquid preparation to the areas of hair loss induced by alopecia areata

Under the same conditions as the experimental example 1, the hair growth agents of the comparative experimental example 1 were applied to the areas of hair loss induced by alopecia areata. However, the agents failed to exhibit any therapeutic effects on alopecia areata by gross observation.

### Experimental example 4 : Skin toxicity test

Since the topical liquid preparation of this invention for promoting hair growth comprises herbal medicine, such as Pinelliae Rhizoma, Caryophylli Flos, Rubi Fructus, Zanthoxyli Fructus, Viticis Fructus, Salviae Radix and Thujae Semen, no adverse reaction or toxicity was observed.

The above test results showed that when the topical liquid preparation according to this invention was applied to the areas of hair loss, the hair loss therein was significantly reduced 15 days after the administration. The topical liquid preparation was proven to be quite effective against the hair loss induced by adverse reaction to drug administration and/or radiological treatment. When the topical liquid preparation was applied to atopic patients, it was effective in the treatment of atopic dermatitis while preventing atopic hair loss. In addition, the preparation was shown to be very effective in the treatment of hair loss induced by alopecia areata.

Since the topical liquid preparation of the invention herein contains herbal medicine as active ingredients, the preparation does not have any side-effects or adverse reaction from the use thereof.

## Claims

1. A topical liquid composition for promoting hair growth, comprising the extracts of Pinelliae Rhizoma, Caryophylli Flos, Rubi Fructus, Zanthoxyli Fructus, Viticis Fructus, Salviae Radix, and Thujae Semen, as active ingredients.

2. A topical liquid composition for promoting hair growth according to claim 1, wherein each of said active ingredients is comprised in an equal weight ratio.

3. A topical liquid composition for promoting hair growth according to claim 1 or 2, wherein the oil extract and ethanol extract containing said active ingredients are mixed in the ratio of 1 : 10 - 15 by weight.

4. A topical liquid composition for promoting hair growth according to claim 3, wherein one or more oil components of said oil extract is selected from the group consisting of sesame oil, perilla oil, and Thujae Semen oil.

5. A process of manufacturing the topical liquid composition for promoting hair growth, wherein 20 - 40 weight parts of the powder comprising Pinelliae Rhizoma, Caryophylli Flos, Rubi Fructus, Zanthoxyli Fructus, Viticis Fructus, Salviae Radix and Thujae Semen are immersed in 100 weight parts of oil and 100 weight parts of ethanol, respectively, and aged for 5 - 10 days, after which oil extract and ethanol extract are mixed in the weight ratio of 1 : 10 - 15.

6. A process of manufacturing the topical liquid composition for promoting hair growth according to claim 5, wherein the powder to be extracted comprises each of said active ingredients in an equal weight ratio.

## Patentansprüche

1. Örtlich aufzubringende flüssige Zusammensetzung zur Förderung des Haarwachstums, die die Extrakte von *Pinelliae rhizoma, Caryophylli flos, Rubi fructus, Zanthoxyli fructus, Viticis fructus, Salviae radix und Thujae semen* als Wirkstoffe umfaßt

2. Örtlich aufzubringende flüssige Zusammensetzung zur Förderung des Haarwachstums nach Anspruch 1, in der jeder dieser Wirkstoffe in einem gleichen Gewichtsverhältnis enthalten ist.

3. Örtlich aufzubringende flüssige Zusammensetzung zur Förderung des Haarwachstums nach Anspruch 1 oder 2, in der der Ölextrakt und der Ethanolextrakt, die diese Wirkstoffe enthalten, im Gewichtsverhältnis von 1 : 10 bis 1 : 15 vermischt werden.

4. Örtlich aufzubringende flüssige Zusammensetzung zur Förderung des Haarwachstums nach Anspruch 3, in welcher ein oder mehrere Ölkomponenten des Ölextraktes aus der Gruppe Sesamöl, Perillaöl und Thujaesamenöl ausgewählt werden.

5. Verfahren zur Herstellung einer örtlich aufzubringenden flüssigen Zusammensetzung zur Förderung des Haarwachstums, worin 20 bis 40 Gewichtsteile des Pulvers, das *Pinelliae rhizoma, Caryophylli flos, Rubi fructus, Zanthoxyli fructus, Viticis fructus, Salviae radix und Thujae semen* umfaßt, in 100 Gewichtsteilen Öl bzw. 100 Gewichtsteile Ethanol eingetaucht und 5 bis 10 Tage gealtert werden, wonach der Ölextrakt und der Ethanolextrakt im Gewichtsverhältnis 1 : 10 bis 1 : 15 vermischt werden.

6. Verfahren zur Herstellung der örtlich aufbringbaren flüssigen Zusammensetzung zur Förderung des Haarwachstums nach Anspruch 5, wobei das zu extrahierende Pulver jedes der Wirkstoffe in einem gleichen Gewichtsverhältnis umfaßt.

## Revendications

1. Composition topique liquide favorisant la pousse des cheveux, comprenant les extraits de Rhizoma Pinelliae, Flos Caryophylli, Fructus Rubi, Fructus Zanthoxyli, Fructus Viticis, Radix Salviae et Semen Thujae, en tant que principes actifs.

2. Composition topique liquide favorisant la pousse des cheveux selon la revendication 1, dans laquelle chacun desdits principes actifs est présent dans un rapport pondéral identique.

3. Composition topique liquide favorisant la pousse des cheveux selon la revendication 1 ou 2, dans laquelle l'extrait huileux et l'extrait éthanolique contenant lesdits principes actifs sont mélangés dans un rapport de 1 : 10 à 15 en poids.

4. Composition topique liquide favorisant la pousse des cheveux selon la revendication 3, dans laquelle un ou plusieurs composants huileux dudit extrait huileux sont choisis dans le groupe constitué par l'huile de sésame, l'huile de périlla et l'huile de Semen Thujae.

5. Procédé de fabrication de la composition topique liquide favorisant la pousse des cheveux, dans lequel 20 à 40 parties en poids de la poudre comprenant Rhizoma Pinelliae, Flos Caryophylli, Fructus Rubi, Fructus Zanthoxyli, Fructus Viticis, Radix Salviae et Semen Thujae sont plongées respectivement dans 100 parties en poids d'huile et 100 parties en poids d'éthanol, puis vieillies pendant 5 à 10 jours, après quoi l'extrait huileux et l'extrait éthanolique sont mélangés dans le rapport pondéral de 1 : 10 à 15.

6. Procédé de fabrication de la composition topique liquide favorisant la pousse des cheveux selon la revendication 5, dans lequel la poudre devant être extraite comprend chacun desdits principes actifs dans un rapport pondéral identique.
